(19) Europäisches Patentamt
European Patent Office
Office européen des brevets

(11) EP 1 731 150 A1

(12) **EUROPEAN PATENT APPLICATION**
published in accordance with Art. 158(3) EPC

(43) Date of publication:
**13.12.2006 Bulletin 2006/50**

(21) Application number: **04717289.5**

(22) Date of filing: **04.03.2004**

(51) Int Cl.:
*A61K 31/409* (2006.01) *A61K 45/06* (2006.01)
*A61P 3/10* (2006.01) *A61P 9/10* (2006.01)
*A61P 25/00* (2006.01) *A61P 29/00* (2006.01)
*A61P 35/00* (2006.01) *A61P 43/00* (2006.01)

(86) International application number:
**PCT/JP2004/002750**

(87) International publication number:
**WO 2005/084665 (15.09.2005 Gazette 2005/37)**

(84) Designated Contracting States:
**AT BE BG CH CY CZ DE DK EE ES FI FR GB GR HU IE IT LI LU MC NL PL PT RO SE SI SK TR**

(71) Applicant: **Yuasa, Makoto**
**Soka-shi,**
**Saitama 340-0021 (JP)**

(72) Inventors:
- **YUASA, Makoto**
  **Soka-shi, Saitama 340-0021 (JP)**
- **OYAIZU, Kenichi**
  **Kamagaya-shi, Chiba 273-0112 (JP)**
- **YAMAGUCHI, Aritomo**
  **Yokohama-shi, Kanagawa 227-0034 (JP)**
- **HANYUU, Yukihiro**
  **Furukawa-shi, Miyagi 989-6175 (JP)**
- **KASAHARA, Kazunori**
  **Gunma-gun, Gunma 370-3511 (JP)**
- **KOMURO, Masayasu**
  **Tsukuki-gun, Kanagawa 220-0203 (JP)**

(74) Representative: **Stuttard, Garry Philip et al**
**Urquhart-Dykes & Lord LLP**
**Tower North Central**
**Merrion Way**
**Leeds LS2 8PA (GB)**

# (54) NIOSOME HAVING METAL PORPHYRIN COMPLEX EMBEDDED THEREIN, PROCESS FOR PRODUCING THE SAME AND DRUG WITH THE USE THEREOF

(57) A niosome having a metalloporphyrin complex embedded therein comprising a cationized metalloporphyrin complex and a niosome-forming substance. The niosome having a metalloporphyrin complex embedded therein has an SOD activity, can interact with superoxide anionic radicals ($O_2^-$) as a target, and can reduce these radicals without fail. The niosome having a metalloporphyrin complex embedded therein can reach cells in living bodies such as cancer cells due to properties of a niosome. Therefore, the niosome having a metalloporphyrin complex embedded therein can exhibit an excellent effect of treating cancer by reducing O2-· in cancer cells. In addition, since the effect is selective, the niosome can be used as a novel anticancer agent without side effects. Moreover, the niosome having a metalloporphyrin complex embedded therein can be retained in the blood while exhibiting a superior antioxidation effect. The niosome can thus protect living bodies from hindrance brought about by active oxygen species.

EP 1 731 150 A1

## Description

### TECHNICAL FIELD

The present invention relates to a niosome having a metalloporphyrin complex embedded therein and, more particularly, to a niosome having a metalloporphyrin complex embedded therein acting as an anticancer agent or an antioxidant in living bodies, and a process for producing the same.

### BACKGROUND ART

Many active oxygen species generated in living bodies are said to participate in many diseases such as inflammation, nervous system diseases, arteriosclerosis, cancer, and diabetes. Usually, in order to maintain a balance, the living bodies produce radical scavenging enzymes such as superoxide dismutase (SOD), catalase, and the like to combat these active oxygen species.

However, cancer cells in living bodies are known to contain a large number of $O_2^-$, radicals. Therefore, enzyme activities are considered to be reduced in the cancer cells.

On the other hand, diseases such as inflammation, nervous system diseases, arteriosclerosis, and diabetes are thought to be caused by an increase in active species such as $O_2^{-\cdot}$ radicals due to imbalanced secretion of radical scavenging enzymes such as SOD, catalase, and the like.

Metalloporphyrin complexes are reported to exhibit high SOD activity. Therefore, if administered to a living body, a metalloporphyrin complex is expected to effectively scavenge active oxygen species such as $O_2^{-\cdot}$ radicals and protect the living body from hindrance brought about by the active oxygen species.

However, since administering a metalloporphyrin complex independently to a living body has many problems from the viewpoint of safety and effects, no metalloporphyrin complexes have been used as a drug.

The present invention has been completed in view of these problems and has an object of providing a means for safely administering a metalloporphyrin complex to a living body and causing the SOD activity possessed by metalloporphyrin complex to be effectively exhibited.

A further object of the present invention is to provide an anticancer agent that can replace anticancer agents such as clinically used cisplatin and mitomycin C causing serious side effects by selectively exhibiting an effect on cancer cells and also to provide an antioxidant for treating diseases other than cancer such as inflammation, nervous system diseases, arteriosclerosis, and diabetes in which active oxygen species are considered to be involved.

### DISCLOSURE OF THE INVENTION

As a result of extensive studies in order to develop a method for decreasing the concentration of $O_2^{-\cdot}$ radicals in cancer cells using the SOD activity of metalloporphyrin complexes, the present inventors have found that the metalloporphyrin complexes can be safely administered while maintaining superior SOD activity and can be retained in the blood by being embedded in a niosome. This finding has led to completion of the present invention.

Specifically, the present invention provides a niosome having a metalloporphyrin complex-embedded therein comprising a cationized metalloporphyrin complex and a niosome-forming substance.

The present invention further provides a process for producing a niosome having a metalloporphyrin complex embedded therein comprising mixing a cationized metalloporphyrin complex and a niosome-forming substance and treating the mixture with supersonic waves in a medium.

The present invention further provides a process for producing a metalloporphyrin complex-embedding niosome comprising reacting a cationized metalloporphyrin complex and an anionic surfactant to produce an ion complex, mixing the resulting ion complex with a niosome-forming substance, and treating the mixture with supersonic waves in a medium.

The present invention also provides a drug comprising the metalloporphyrin complex-embedding niosome as an effective component.

### BRIEF DESCRIPTION OF THE DRAWING

Figure 1 shows results of tests for confirming an anticancerous effect using FeT2MPyP as a metalloporphyrin complex and Pluronic F-88 as a niosome-forming substance, wherein the solid line connecting black triangle marks indicates the result of ion complex 2 (FeT2MPyP/4SAS), the dotted line connecting black square marks indicates the result of Pluronic F-88, solid line connecting black square marks indicates the result of CDDP, the solid line connecting black diamond marks indicates the result of MMC, and the solid line connecting black circle marks indicates the result of the invention product 9.

BEST MODE FOR CARRYING OUT THE INVENTION

In the present invention, "metalloporphyrin complex-embedding niosome" indicates a niosome in which a metalloporphyrin complex is incorporated, formed by a niosome-forming substance such as a mixture of a nonionic surfactant, a cholesterol, and the like, in which either a part of the metalloporphyrin complex may be present outside the niosome membrane or the entire amount of the metalloporphyrin complex is embedded in the niosome membrane.

The metalloporphyrin complex-embedding niosome of the present invention comprises a cationized metalloporphyrin complex and a niosome-forming substance.

The cationized metalloporphyrin complex of the metalloporphyrin complex-embedding niosome (hereinafter referred to from time to time as "Por-embedding niosome") of the present invention is a porphyrin complex having a group containing a cationic nitrogen atom as a substituent and includes, for example, the following compounds (I), (II), and (III),

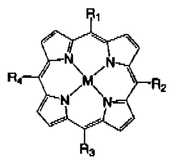
(I)

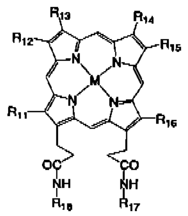
(II)

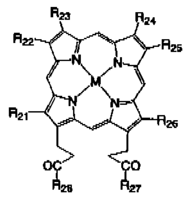
(III)

wherein at least one of $R_1$ to $R_4$ is a group selected from an N-lower-alkylpyridyl group, a lower-alkylammoniophenyl group, and an N-lower-alkylimidazolyl group, $R_{11}$ to $R_{16}$ and $R_{21}$ to $R_{26}$ are lower alkyl or lower alkoxy groups, $R_{17}$ and $R_{18}$ are groups selected from an N-lower-alkylpyridyl group, a lower-alkylammoniophenyl group, and an N-lower-alkylimidazolyl group, and $R_{27}$ and $R_{28}$ indicate N-alkylammonio groups.

Compounds having a methylpyridyl group for $R_1$ in the above formula (I) such as 5,10,15,20-tetrakis(2-methylpyridyl)porphyrin (T2MPyP), 5,10,15,20-tetrakis(4-methylpyridyl)porphyrin (T4MPyP), 5,10,15,20-tetrakis(3-methylpyridyl)porphyrin (T3MPyP), and the like, compounds having a methylpyridylamideethyl group for $R_2$ in the above formula (II) such as [1,3,5,8-tetramethyl-2,4-divinyl-6,7-di(4-methylpyridylamideethyl)]porphyrin (PPIX-DMPyAm) and the like can be given as more specific examples.

As the metal coordinating to these cationized porphyrin complexes, iron (Fe), manganese (Mn), cobalt (Co), copper (Cu), molybdenum (Mo), chromium (Cr), iridium (Ir), and the like are preferable.

Among the above-mentioned metalloporphyrin complexes, the cationized porphyrin complex coordinated with a metal shown by the formula (I) can be synthesized according to a method described in K. Kalyanasundaram, Inorg. Chem., 23, 2453 (1984), A. D. Adler et al., J. Inorg. Nucl. Chem., 32, 2443 (1970), T, Yonetani et al., J. Biol. Chem., 245, 2988 (1970), P. Hambright, Inorg. Chem., 15, 2314 (1976), and the like.

The cationized porphyrin complex shown by the formula (II) coordinated with a metal can be synthesized according to a method described in E. Tsuchida, H. Nishide, H. Yokoyama, R. Young, and C. K. Chang, Chem. Lett., 1984, 991, and the like.

The Por-embedding niosome of the present invention can be produced by using only the above-mentioned cationized porphyrin complexes, but more preferably is produced by using an ion complex obtained by reacting a cationized metalloporphyrin complex with an anionic surfactant (hereinafter referred to simply as "ion complex").

As examples of the anionic surfactant used for producing the ion complex, alkali metal salts of a fatty acid and alkali metal salts of alkyl sulfuric acid are preferable. As specific examples, alkali metal salts of a fatty acid such as lauric acid (LAS), myristic acid (MAS), palmitic acid (PAS), stearic acid (SAS), and oleic acid (OAS), and alkali metal salts of an alkyl sulfuric acid such as dodecylsulfuric acid (SDS), tetradecylsulfuric acid (STS), hexadecylsulfuric acid (SHS), octadecylsulfuric acid (SOS), and the like can be given. Sodium, potassium, and the like are preferable as the metal components of the alkali metal salts of a fatty acid and alkyl sulfuric acid.

In order to form the above ion complex, a cationized metalloporphyrin complex is mixed with an anionic surfactant in a suitable solvent at a molar ratio of the cationized metalloporphyrin complex to the anionic surfactant of from about 1:1 to 1:20.

On the other hand, the above cationized metalloporphyrin complex or the ion complex made from the cationized metalloporphyrin complex and the anionic surfactant is mixed with a niosome-forming substance to produce a Por-embedding niosome by a conventional method of producing a niosome.

As the niosome-forming substance, many compounds reported to produce a niosome can be used. Nonionic

surfactants and mixtures of a nonionic surfactant and a cholesterol or triacylglycerol are preferably used.

As the nonionic surfactant, polyoxyethylene sorbitan fatty acid esters, sorbitan fatty acid esters, polyoxyethylene polyoxypropylene copolymers, and the like can be used either individually or in combination. As specific examples of the nonionic surfactant, Tween-61 (TW61), Tween-80, and Span 80 (manufactured by NOF Corp.), Pluronic F-88 (F88) (manufactured by ADEKA Corp.), and the like can be given.

As examples of the cholesterol, cholesterol, α-cholestanol, β-cholestanol, cholestane, desmosterol (5,24-cholestadiene-3β-ol), sodium cholate, and cholecalciferol can be given.

In order to produce a Por-embedding niosome from the above cationized metalloporphyrin complex or the ion complex and the niosome-forming substance, these components must first be added to a suitable solvent and sufficiently mixed.

In forming a niosome, the cationized metalloporphyrin complexes shown by the formulas (II) or (III) can be embedded in the niosome as is, but the cationized metalloporphyrin complex shown by the formulas (I) is preferably embedded into a niosome in the form of an ion complex.

The amount of the cationized metalloporphyrin complex and the niosome-forming substance used in forming the niosome, in terms of the amount (mol) of the niosome-forming substance for one mol of the cationized metalloporphyrin complex or the ion complex, is preferably from 10 to 500 mols, and particularly preferably from 50 to 300 mols.

The niosome can be formed by a commonly known process, for example, a process comprising dissolving and mixing the above components in a volatile solvent, removing only the volatile solvent by vaporization, dissolving the residue in an appropriate aqueous solvent such as purified water or a physiological saline solution, and vigorously stirring or supersonically treating the solution to form a Por-embedding niosome.

If necessary, a solution of a pharmaceutically effective component or a certain type of culture medium may be used instead of the aqueous solvent to produce a Por-embedding niosome in which such a pharmaceutically effective component or a culture medium is incorporated.

As a result of structural analysis of the Por-embedding niosome obtained in this manner using a fluorescence spectrum, dynamic light scattering measurement, and the like, as described later, it was confirmed that a cationized metalloporphyrin complex portions are present on the surface of the niosome or in hydrophilic areas oflipids and the like and alkyl group side chains ofthe surfactant are embedded in hydrophobic areas of lipids.

It was also confirmed that the niosome has a particle size of 100 nm or less, and particularly can be made into endoplasmic reticula with a diameter of 30 nm or less, which is a size enabling the niosome to reach cells when incorporated into the body.

In addition, as a result of the test for confirming the anticancerous effect of the Por-embedding niosome as described later, the Por-embedding niosome was confirmed to be more effective than a simple cationized metalloporphyrin complex used as a raw material of the niosome. The effect was confirmed to be higher than the effect of cisplatin and mitomycin C which are presently used as anticancer agents.

Moreover, the evaluation ofthe SOD activity of the Por-embedding niosome confirmed that the Por-embedding niosome exhibits an SOD activity equivalent to the simple cationized metalloporphyrin complex used as a raw material of the niosome, indicating usability as an SOD compound possessing superior retainability in the blood.

As mentioned above, the Por-embedding niosome of the present invention has excellent anticancer activity and can be used as an anticancer agent in the field of cancer therapy.

Therefore, it is possible to treat cancer of a patient by administering the Por-embedding niosome to the patient by direct dosage, intravenous administration, subcutaneous administration, and the like.

The Por-embedding niosome of the present invention also possesses a superior effect as an antioxidant and can be used for protecting living bodies from disorders brought about by active oxygen species such as, for example, inflammation, nervous system disorders, arteriosclerosis, and diabetes.

Therefore, if the Por-embedding niosome is administered to a patient suffering from inflammation, nervous system disorders, arteriosclerosis, or diabetes by direct dosage, intravenous administration, subcutaneous administration, or the like means, that patient as cancer can be treated.

EXAMPLE

The present invention will be described in more detail by way of Examples and Test Examples which should not be construed as limiting the present invention.

Example 1

Synthesis of iron[5,10,15,20-tetrakis(2-methylpyridyl)porphyrin] (FeT2MPyP)

(1) After heating 500 ml of propionic acid to 110°C with stirring, 15 ml (0.158 mol) of 2-pyridylcarboxyaldehyde was added. Then, 12 ml (0.173 mol) of pyrrole was slowly added dropwise using an injector and the mixture was refluxed at 110°C for one hour to effect a cyclization-condensation reaction. After the reaction, the reaction solution was allowed to cool to room temperature and the solvent was evaporated. The residue was neutralized, washed, and purified by column chromatography (alumina basic type-I, chloroform) to obtain 5,10,15,20-tetrakis(2-pyridyl)porphyrin (amount: 1.1 g, yield: 4.4%).
$^1$H-NMR $\delta$H($CDCl_3$, ppm): -2.82 (H of pyrrole NH, 2H), 7.72-9.14 (H of pyridine, 16H), 8.87 (H ofpyrrole, 8H)
UV-vis $\lambda$max (chloroform, nm): 418, 513, 544, 586, 645
FAB-Mass(m/z): 619, 620

(2) Next, 0.2 g ($3.2\times 10^{-4}$ mol) of 5,10,15,20-tetrakis(2-pyridyl)porphyrin obtained in (1) above was added to and dissolved in 150 ml of dimethylformamide under an argon (Ar) atmosphere. After the further addition of iron bromide ($FeBr_2$) produced from 0.2 g of iron and 5 ml of 48% hydrobromic acid, the mixture was refluxed for four hours. After the reaction, the reaction solution was allowed to cool to room temperature and the solvent was evaporated.
The residue was purified by fractionation using a water-chloroform mixture, followed by column chromatography (alumina basic type-I, methanol) to obtain iron[5,10,15,20-tetrakis(2-pyridyl)porphyrin] (amount: 0.21 g, yield: 94%) which is a precursor of the target compound.
UV-vis $\lambda$max (methanol, nm): 408, 512, 566
FAB-Mass(m/z): 672

(3) 0.1 g of iron[5,10,15,20-tetrakis(2-pyridyl)porphyrin] obtained in (2) above and 6 ml of methyl p-toluenesulfonate were added to 30 ml of dimethylformamide and the mixture was refluxed at 130°C for five hours. After refluxing, the reaction solution was allowed to cool to room temperature and the solvent was evaporated. The residue was purified by extraction and column chromatography (alumina basic type-I, methanol) to obtain the target compound FeT2MPyP (yield: 91%).
UV-vis $\lambda$max (water, nm): 408, 584
Elemental analysis (%): Found: C75.11, H3.97, N17.66, C/N4.25
Calcd: C77.58, H4.24, N18.12, C/N4.28

(4) Iron[5,10,15,20-tetrakis(4-methylpyridyl)porphyrin] (FeMT4MPyP) was obtained in the same manner as in the steps (1) to (3) above except for using 4-pyridylcarboxyaldehyde instead of 2-pyridylcarboxyaldehyde in the step (1). Other metalloporphyrin complexes of [5,10,15,20-tetrakis(4-methylpyridyl)porphyrin] (MT4MPyP) and [5,10,15,20-tetrakis(2-methylpyridyl)porphyrin] (MT2MPyP) can be synthesized in the same manner as above.

Example 2

Synthesis of manganese[5,10,15,20-tetrakis(2-methylpyridyl)porphyrin] (MnT2MPyP)

(1) After heating 500 ml of propionic acid to 110°C, 15 ml of 2-pyridylcarboxyaldehyde was added, following which 12 ml of pyrrole was slowly added. After the addition of all the amounts, the mixture was refluxed for one hour. After the reaction, the reaction mixture was allowed to cool, evaporated, neutralized, and washed. The residue was purified by column chromatography (basic alumina, chloroform) and the like to obtain purple crystals of 5,10,15,20-tetrakis (2-pyridyl)porphyrin (amount: 1.68 g, yield: 7.08%),
$^1$H-NMR $\delta$H($CDCl_3$, ppm): -2.8 (H of pyrrole NH, 2H), 7.7 (H of pyridine (4 position), 4H), 8.1 (H of pyridine (5 position), 4H), 8.2 (H of pyridine (6 position), 4H), 9.2 (H of pyridine (4 position), 4H), 8.8 (H of pyrrole, 8H)
UV-vis $\lambda$max (chloroform, nm): 418, 513, 544, 586, 645
FAB-Mass(m/z): 619,620

(2) 1.98 g of manganese acetate tetrahydrate was added to 200 ml of a dimethylformamide (DMF) solution containing 500 mg of 5,10,15,20-tetrakis(2-pyridyl)porphyrin obtained in (1) after argon replacement, and the mixture was refluxed for three hours in an argon stream. After the reaction, the reaction mixture was allowed to cool and subjected to evaporation, fractionation with water/chloroform, and drying under reduced pressure to obtain manganese [5,10,15,20-tetrakis(2-pyridyl)porphyrin] (amount: 409 mg, yield: 75.2%).
UV-vis $\lambda$max (chloroform, nm): 461, 555
FAB-Mass(m/z): 672

(3) Next, 200 mg of the above manganese[5,10,15,20-tetrakis(4-pyridyl) porphyrin] was reacted with 12 ml of methyl p-toluenesulfonate at 130°C for eight hours. After the reaction, the reaction mixture was allowed to cool and subjected to fractionation with water/chloroform and column chromatography ((1) acidic alumina and (2) basic alumina, methanol) to obtain the target compound MnT2MPyP (amount: 153 mg).
UV-vis $\lambda$max (water, nm): 456, 555

(4) Manganese[5,10,15,20-tetrakis(4-methylpyridyl)porphyrin] (MnT4MPyP) was obtained in the same manner as in the steps (1) to (3) above except for using 4-pyridylcarboxyaldehyde instead of 2-pyridylcarboxyaldehyde in the step (1). Other metalloporphyrin complexes of [5,10,15,20-tetrakis(2-methylpyridyl)porphyrin] (MT2MPyP) and [5,10,15,20-tetrakis(4-methylpyridyl)porphyrin] (MT4MPyP) can be synthesized in the same manner as above.

Example 3

Synthesis of Por-embedding niosome (1)

(1) 1.4 mg (1 µmol) of FeT2MPyP, a cationized metalloporphyrin complex prepared in Example 1, and 0.3 mg (1 µmol) of SAS, a surfactant, were put into a test tube and 10 ml of methanol was added as a solvent and mixed to obtain an ion complex 1 (FeT2MPyP/1SAS).
An ion complex 2 (FeT2MPyP/4SAS) and ion complex 3 (FeT2MPyP/4OAS) were also prepared in the same manner using 1.2 mg (4 µmol) of SAS and 1.2 mg (4 µmol) of OAS respectively instead of 1 µmol of SAS.
In the same manner, ion complexes 4 to 9 shown in Table 1 were prepared using MnT2MPyP, MnT4MPyP, FeT2MPyP + MnT2MPyP (0.5 µmol: 0.5 µmol), and FeT4MPyP + MnT4MPyP (0.5 µmol : 0.5 µmol) as cationized metalloporphyrin complexes and SAS or OAS as surfactants.

Table 1

| Ion complex | Cationized metalloporphyrin | Surfactant | Cationized metalloporphyrin/Surfactant |
|---|---|---|---|
| 1 | FeT2MPyP [1 µmol] | SAS [1 µmol] | 1/1 |
| 2 | FeT2MPyP [1 µmol] | SAS [4 µmol] | 1/4 |
| 3 | FeT2MPyP [1 µmol] | OAS [4 µmol] | 1/4 |
| 4 | MnT4MPyP [1 µmol] | SAS [1 µmol] | 1/1 |
| 5 | MnT4MPyP [1 µmol] | SAS [4 µmol] | 1/4 |
| 6 | MnT2MPyP [1 µmol] | SAS [1 µmol] | 1/1 |
| 7 | MnT2MPyP [1 µmol] | SAS [4 µmol] | 1/4 |
| 8 | FeT2MPyP + MnT2MPyP (1:1) [1 µmol] | SAS [4 µmol] | 1/4 |
| 9 | FeT4MPyP + MnT4MPyP (1:1) [1 µmol] | SAS [4 µmol] | 1/4 |

(2) Next, the above ion complex 1 (1 µmol), 0.131 g (100 µmol) of Tween-61, a niosome-forming substance, and 0.0386 g (100 µmol) of cholesterol were put into a test tube, to which a small amount of chloroform as a solvent was added and mixed. After evaporation of the solvent to form a thin film, 10 ml of a physiological saline solution was added and the mixture was supersonically treated in an ice cooled bath under an argon atmosphere for 3 minutes using a probe-type supersonic-wave irradiation apparatus at 30 W. After the supersonic treatment, the product was allowed to stand for 30 minutes at 60°C and sterilized by filtration using a 0.22 µm diam. filter to obtain a Por-embedding niosome (FeT2MPyP/1SAS-embedding TW61-chol niosome; Invention Product 1).
(3) Another Por-embedding niosome (FeT2MPyP/4SAS-embedding TW61-chol niosome; Invention Product 2) was obtained in the same manner as in (2) above using the ion complex 2 (1 µmol). Por-embedding niosomes (Invention Products 3 to 8) shown in Table 2 can be obtained in the same manner using the ion complexes 1, 2, and 4 to 6.

Table 2

| Ribosome having porphyrin complex embedded therein | Ion complex | Niosome-producing substance |
|---|---|---|
| FeT2MPyP/1SAS-embedding TW61-chol niosome (Invention Product 1) | 1 | Tween-61 and cholesterol (1:1) |
| FeT2MPyP/4SAS-embedding TW61-chol niosome (Invention Product 2) | 2 | Tween-61 and cholesterol (1:1) |
| FeT4MPyP/1SAS-embedding TW61-chol niosome (Invention Product 3) | 1 | Tween-61 and cholesterol (1:1) |
| FeT4MPyP/4SAS-embedding TW61-chol niosome (Invention Product 4) | 2 | Twccn-61 and cholesterol (1:1) |
| MnT2MPyP/ISAS-embedding TW61-chol niosome (Invention Product 5) | 6 | Tween-61 and cholesterol (1:1) |
| MnT2MPyP/4SAS-embedding TW61-chol niosome (Invention Product 6) | 7 | Tween-61 and cholesterol (1:1) |
| MnT4MPyP/1SAS-embedding TW61-chol niosome (Invention Product 7) | 4 | Tween-61 and cholesterol (1:1) |
| MnT4MPyP/4SAS-embedding TW61-chol niosome (Invention Product 8) | 5 | Tween-61 and cholesterol (1:1) |

Example 4

Synthesis of Por-embedding niosome (2)

The ion complex 2 (0.5 μmol) shown in Table 1, 0.54 g (50 μmol) of Pluronic F-88, a niosome-forming substance, and 0.0154 g (40 μmol) of cholesterol were put into a test tube, to which a small amount of chloroform (about 3 ml) as a solvent was added and mixed. After evaporation of the solvent to form a thin film, 10 ml of a physiological saline solution was added and the mixture was supersonically treated in an ice cooled bath for 30 minutes using a probe-type supersonic-wave irradiation apparatus at 30 W. After the supersonic treatment, the product was allowed to stand for 30 minutes at 50°C and sterilized by filtration using a 0.22 μm diam. filter to obtain a Por-embedding niosome (FeT2MPyP/4SAS-embedding F88-chol niosome; Invention Product 9).

Example 5

Measurement ofPor-embedding niosome dynamic light scattering (1)

Dynamic light scattering ofPor-embedding niosome was measured using a particle sizing system ("Nicomp 370") in order to determine the particle size and particle size distribution of the Por-embedding niosome. The results of measuring dynamic light scattering of Fe T2MPyP/1SAS-embedding TW61-chol niosome (Invention Product 1) and FeT2MPyP/4SAS-embedding TW61-chol niosome (Invention Product 2) synthesized according to Example 3 are shown in Table 3.

Table 3

| Niosome having porphyrin complex embedded therein | | First distribution peak | | Second distribution peak | |
|---|---|---|---|---|---|
| | | Particle size (nm) | Distribution ratio (%) | Particle size (nm) | Distribution ratio (%) |
| FeT2MPyP/1SAS-embedding TW61-chol niosome (Invention Product 1) | Number distribution | 28.4 | 90 | 145 | 10 |
| FeT2MPyP/4SAS-embedding TW61-chol niosome (Invention Product 2) | Number distribution | 28.5 | 90 | 146 | 10 |

As a result, it can be seen that the number distribution 90% of particles had an average particle size of 28.5 nm and 10% had an average particle size of 146 nm. The results coincided with the results of the TEM inspection. It can also be seen from Table 4 that all Por-embedding niosomes had an average particle size of 100 nm or less, which, when administered, is small enough to pass through microvascular endothelium and reach the target cells.

Example 6

Measurement ofPor-embedding niosome dynamic light scattering (2)

Dynamic light scattering of MnT2MPyP/1SAS-embedding TW61-chol niosome (Invention Product 5) was measured in the same manner as in Example 6. As a result, the niosome was found to have an average particle size of 28.5 nm (the distribution ratio: 90%, the balance of about 10% had an average particle size of 146 nm). The average particle size of MnT2MPyP/4SAS-embedding TW61-chol niosome (Invention Product 6) was 28.5 nm (the distribution ratio: 90%, the balance of about 10% had an average particle size of 146 nm). Both niosomes had an average particle size of 100 nm or less which is small enough to cause no problems when administered to a living body.

Example 7

Fluorescence spectrum measurement of Por-embedding niosome

(1) Fluorescence spectrum measurement ofPor-embedding niosome was conducted using an "RF-5300PC" (manufactured by Shimadzu Corp.) in order to determine the place at which the embedded porphyrin complex is present in the niosome.

In the measurement, since a metalloporphyrin complex extinguishes fluorescence in general, cationized metal-free porphyrin complexes (hereinafter referred to as "metal-free complex") were synthesized according to the methods of Examples 1, 2, etc. and used instead of the metalloporphyrin complexes as fluorescence probes. Metal-free complex-embedding niosomes were synthesized according to Example 3. Hereinafter, as abbreviation of a metal-free complex, $H_2$ is used instead of M in the formula of the cationized metalloporphyrin complex. For instance, the metal-free complex of metal[5,10,15,20-tetrakis(2-methylpyridyl)porphyrin] (MT2MPyP) is indicated as $H_2$T2MPyP, and the metal-free complex of metal[5,10,15,20-tetrakis(4-methylpyridyl)porphyrin] (MT4MPyP) is indicated as $H_2$T4MPyP.

A fluorescence spectrum of cationized metal-free complexes-embedding niosome prepared in the above-described

manner, for example, a fluorescence spectrum of$H_2$T2MPyP of various solutions containing $H_2$T2MPyP/4SAS-embedding TW61-chol niosome and the $H_2$T2MPyP/4SAS was measured at a wavelength in a range from 500 to 800 nm. As a result, a fluorescence spectrum having a peak at 645 nm was obtained using an aqueous solution containing $H_2$T2MPyP/4SAS-embedding TW61-chol niosome.
This fluorescence peak wavelength was the middle of the fluorescence wavelengths of $H_2$T2MPyP in water and methanol (fluorescence peak wavelength (short wavelength side) water <embedding niosome solution <metahol (long wavelength side)).
The fluorescence spectra of the $H_2$T2MPyP in various solutions such as methanol (peak: 642 nm), ethanol (peak: 643 nm), 2-propanol (peak: 645 nm), n-butanol (peak: 645 nm), ethylene glycol (peak: 642 nm) had the same waveform as above. However, the fluorescence spectra of $H_2$T2MPyP in water has a broad peak, exhibited a significant decrease in the strength, and shifted to a short wavelength side (630 nm). Based on these results, the $H_2$T2MPyP embedded in niosome is believed to be present in polar conditions resembling the conditions ofthe above alcohols, specifically, near the hydrophilic part of bimolecular membrane of which the conditions are slightly non-polar (slightly more hydrophobic) than the conditions of water. The result of $H_2$T2MPyP/1SAS-embedding TW61-chol niosome was also the same.
(2) Next, fluorescence spectra were measured using 8-anilino-1-naphthalenesulfonic acid (ANS), which is a fluorescence probe being present near the hydrophilic part of the niosome bimolecular membrane and acting as an indicator for the polarity, fluidity, and the like of the bimolecular membrane. All fluorescence spectra of ANS in methanol, methanol/chloroform, and a solution of ANS-embedding TW61 niosome (no porphyrin complex is embedded) were similar to each other having a peak at 485nm (excitation wavelength: 385 nm). Next, a fluorescence spectrum of ANS was measured in a solution of TW61 niosome in which a porphyrin complex and ANS are embedded together. The spectrum did not have a peak at 485 nm, but showed two peaks at 450 nm and 500 nm, each having a peak fluorescence strength weaker than the peak fluorescence strength at 485 nm. The presence of Soret band absorption peaks of porphyrin complex in this region is believed to be the result of interaction between ANS and the porphyrin complex, which indicates the presence of the porphyrin complex near the ANS. Based on these results, the $H_2$T2MPyP embedded in niosome is believed to be present in polar conditions resembling the conditions of the above alcohols, specifically, near the hydrophilic part of the bimolecular membrane.

In a niosome in which the porphyrin complex is embedded together with 1,6-diphenyl-1,3,5-hexatriene (DPH), which is a fluorescent probe existing in the inside of a bimolecular membrane (hydrophobic part), no peak reduction was seen in the Soret band of porphyrin complex and in DPH peaks at 410 nm, 434 nm, and 457 nm, indicating that there was no interaction between DPH and the porphyrin complex and that the porphyrin complex did not exist near the DPH. Based on these results, the $H_2$T2MPyP embedded in niosome is believed to be present in polar conditions resembling the conditions of the above alcohols, specifically, near the hydrophilic part of the bimolecular membrane.

Example 8

Fluorescence depolarization measurement of Por-embedding niosome

Fluorescence depolarization of a metalloporphyrin complex-embedding niosome was measured using 1,6-diphenyl-1,3,5-hexatriene (DPH, 1 $\mu$M) existing near the hydrophilic part of a bimolecular membrane as a fluorescent probe (instrument: polarization auxiliary analyzer for an RF-5300PC and RF-540/5000 manufactured by Shimadzu Corp., excitation wavelength: 361 nm, fluorescence wavelength: 428 nm) at 5-60°C. In the relation of the fluorescence depolarization measurement temperature and the degree of polarization (a reverse sigmoidal curve) of the TW61-chol niosome containing DPH (blank), for example, a reduction of the fluorescence polarization degree was seen near the phase transition temperature (Tc = 36°C) of the TW61-chol niosome bimolecular membrane. In the relation of the fluorescence depolarization measurement temperature and the degree of polarization of the FeT2MPyP/1SAS-embedding TW61-chol niosome containing DPH, although a reduction of the fluorescence polarization degree was seen near the phase transition temperature (Tc = 37°C) in the same manner as in the blank, the polarization degree was large in a gel state. This is due to the interaction among FeT2MPyP/1SAS, Tween-61, and cholesterol, indicating that FeT2MPyP/1SAS is present in the niosome bimolecular membrane formed by Tween-61 and cholesterol.
The relation of the fluorescence depolarization measurement temperature and the degree of polarization of the MnT2MPyP/1SAS-embedding niosome containing DPH was seen to slightly shift to a high temperature side as compared with the blank and the polarization degree along the vertical axis increased in a gel state. These results are based on the interaction among MnT2MPyP/1SAS, Tween-61, and cholesterol and support the presence of MnT2MPyP/1SAS in the niosome bimolecular membrane formed by Tween-61 and cholesterol.

Example 9

Test of anticancer properties of Por-embedding niosome

The anticancer properties of the Por-embedding niosome of the present invention were examined by a cytotoxic test (cell-death test) according to the Alamar Blue method.

Various Por-embedding niosomes (metalloporphyrin complex concentrations: 0, 12.5, 25, 50, and 100 μM) were used as the test samples. As reference samples, metalloporphyrin complexes (concentrations: 0, 12.5, 25, 50, and 100 μM) and niosomes (concentrations: 2,500, 5,000, 10,000, and 20,000 μM) which are respectively components of the Por-embedding niosomes were used. Both concentrations correspond to the Por-embedding niosome concentrations.

As comparative samples, currently used anticancer agents such as cisplatin (CDDP, concentration: 0, 10, 20, 40, 80 μM), mitomycin C (MCC, concentration: 0, 7.5, 15, 30, 60 μM), and the like were used with mouse lung cancer cells (Lewis Lung Carcinoma (LLC) supplied by RIKEN Gene Bank).

A specific test method was as follows. Cells were cultured in a DMEM medium to which 10% FBS was added, followed by counting of the number of cells and adjusting the cell concentration to obtain a cell suspension liquid (100 μl/well, number of cells: $1 \times 10^4$ cells/well). The cell suspension liquid was added to wells in a 96 well plate and the cells were cultured in a $CO_2$ incubator (5% $CO_2$) for 24 hours. After removing the medium from the well plate, previously prepared test sample solutions with a different concentration (active component concentration: 0 to 100 μM) were added in an amount of 100 μl/well, followed by incubation for 24 hours in a $CO_2$ incubator. After the addition of an alamar blue solution (10 μl/well) sterilized by filtration, the mixture was incubated for a further five hours. Then, absorbance (at a wavelength of 570 nm and a reference wavelength of 600 nm) was measured using a microplate reader.

The results obtained by using Pluronic F-88 niosome (Invention Product 9) in which FeT2MPyP was embedded as a Por-embedding niosome are shown in Figure 1. Based on the results, it can be seen that the LLC cell survival rate decreases as the test solution concentration increases, indicating effective anticancer properties. In particular, the LLC cell survival rate when an ion complex of FeT2MPyP/4SAS was used at an FeT2MPyP concentration of 25 μM or more was slightly lower than the rate achieved by CDDP or MMC. In addition, the LLC cell survival rate decreased as the concentration of the FeT2MPyP/4SAS-embedding F88-chol niosome added increased, specifically to as low as 10% or less at an addition concentration of 50 μM, indicating anticancer properties superior to those of the currently-used CDDP and MMC.

The FeT2MPyP/1SA5-embedding TW61-chol niosome (Invention Product 1) and FeT2MPyP/4SAS-embedding TW61-chol niosome (Invention Product 2), both of which are Por-embedding niosomes, also showed anticancer properties superior to those of the currently-used CDDP and MMC. When the concentration was 50 μM, for example, the anticancer effect increased in the order of currently-used anticancer agent < ion complex < cationized metalloporphyrin complex < metalloporphyrin complex-embedding niosome. The metalloporphyrin complex-embedding niosome can thus be concluded to be an excellent anticancer agent.

Example 10

Interaction between metalloporphyrin complex and hydrogen peroxide ($H_2O_2$)

A metalloporphyrin complex with a low molecular weight is reported to be easily decomposed in the presence of a large excess amount of hydrogen peroxide ($H_2O_2$) due to a high probability of porphyrin rings to interact with $H_2O_2$ because the porphyrin rings are exposed (R. F. Pasternack and B. Halliwell, J. Am. Chem. Soc., 101, 1026 (1979). For this reason, the interaction of a low-molecular weight MnT2MPyP and a niosome (high-molecular weight) MnT2MPyP/1SAS-embedding TW61-chol niosome (Invention Product 5) and MnT2MPyP/4SAS-embedding TW61-chol niosome (Invention Product 6) with $H_2O_2$ was examined by UV-vis measurement. Specifically, these niosomes were interacted with $H_2O_2$ at different concentrations to measure a decay curve of the porphyrin complex Soret band absorption peak (456 nm) based on the decomposition due to the interaction. The reaction rate constant ($k_{H2O2}$) was calculated from the decay curve. The interaction was then evaluated based on the half-life ($t_{1/2}$) calculated from the reaction rate constant. The results obtained by using copper/zinc superoxide dismutase (Cu/Zn-SOD) are also shown for reference. In the experiment, the samples were prepared according to the method described in Example 2, in which $H_2O_2$ concentrations of 1.2, 2.0, 3.0, and 30 mM, and a metalloporphyrin complex concentration of 5 μM were used. The results are shown in Table 4.

Table 4

| Metalloporphyrin complex system | $t_{1/2}$ (sec) |
|---|---|
| MnT2MPyP | 730 |
| MnT2MPyP/1SAS-embedding TW61-chol niosome (Invention Product 5) | 1,005 |
| MnT2MPyP/4SAS-embedding TW61-chol niosome (Invention Product 6) | 806 |
| Cu/Zn-SOD (comparative substance) | 10 |

The results show that the $t_{1/2}$ increased in the order of Cu/Zn-SOD < MnT2MPyP < MnT2MPyP/4SAS-embedding TW61-chol niosome < MnT2AfPyP/1SAS-embedding TW61-chol niosome, indicating that the MnT2MPyP having exposed porphyrin rings due to the low molecular weight has a large probability of interacting with $H_2O_2$ and is easily decomposed, whereas MnTM2PyP-embedding TW61-chol niosomes having a higher molecular weight are decomposed only with difficulty because porphyrin rings are not exposed due to the high molecular weight resulting in a low probability of interacting with $H_2O_2$. The results coincided with the results of the fluorescence spectrum measurement in Example 7. In addition, it was shown that the $t_{1/2}$ of the MnT2MPyP/1SAS-embedding TW61-chol niosome is greater than the $t_{1/2}$ of Cu/Zn-SOD, indicating that the MnT2MPyP/ISAS-embedding TW61-chol niosome has greater $H_2O_2$ resistance than Cu/Zn-SOD.

Example 11

Evaluation of SOD activity of Por-embedding niosome (1)

The SOD activity (i.e. $O_2^{-\cdot}$ extinction activity) of Por-embedding niosome was evaluated according to the cytochrome c method ((1) J. M. Mccord and I. Fridovich, J. Biol. Chem., 244, 6049 (1969) and (2) J. Butler, W. H. Kopenol, E. Margoliash, J. Biol. Chem., 257, 10747 (1982)). A specific procedure was as follows: Five or more Por-embedding niosome solutions (A solutions) with different metalloporphyrin concentrations ranging from 0 to 1,000 μM were prepared. A mixed solution (B solution) was prepared by mixing a 0.3 mM xanthine aqueous solution, a 60 μM cytochrome c aqueous solution, and a 30 mM phosphoric acid buffer solution (7.8 pH), each in an amount of 20 ml, and adding 24 ml of purified water. 0.3 ml of A solution and 0.2 ml of purified water were added to 2.1 ml of B solution and the resulting mixture was allowed to stand at 25°C for 10 minutes. Then, 0.1 ml of a 7 μg/ml catalase aqueous solution and 5 μl of a 25 U/ml xanthine oxidase (XOD) aqueous solution were rapidly added to and mixed with the above mixture after standing to measure UV-vis at 550 nm (an absorption peak based on ferrocytochrome c formation) over time. The ultimate concentrations of the components in the test solutions were 0 to 100 μM of the metalloporphyrin complex, 0.05 mM of xanthine, 2.5 U/ml of XOD, 10 μM of cytochrome c, and 0.23 μg/ml of catalase. The UV-vis was also measured for a solution without addition of Por-embedding niosome (blank) in the same manner.

The ferrocytochrome c formation rates in a solution not containing Por-embedding niosome (vo) and a solution containing Por-embedding niosome (vi) were determined from the relationship between the time and the absorbance at 550 nm in the UV-vis measurement over time. The inhibition coefficient (IC) was calculated according to the following formula to determine the concentration of metalloporphyrin when IC = 50% ($IC_{50}$) from the relationship between the concentration of metalloporphyrin complex and IC. The resulting $IC_{50}$ value was regarded as the SOD activity. The smaller the $IC_{50}$ value, the higher the SOD activity. The SOD activity of MnT4MPyP and the like was evaluated in the same manner for reference.

$$\text{Inhibition coefficient (IC)} = 1 - (vi/vo)$$

vo: rate of formation of ferrocytochrome c in a solution not containing Por-embedding niosome.
vi: rate of formation of ferrocytochrome c in a solution containing Por-embedding niosome

Table 5 shows $IC_{50}$ values for various metalloporphyrin complex systems. In the Table, $IC_{50}$ value of MnT4MPyP is a value disclosed by Fridovich et al. (I. Batinic-Haberle, L. Benov, and I. Fridovich, J. Biol. Chem., 273, 24251 (1998).

Table 5

| Metalloporphyrin complex system | $IC_{50}$ (μM) |
|---|---|
| MnT2MPyP/ISAS-embedding TW61-chol niosome (Invention Product 5) | 0.32 |
| MnT2MPyP/4SAS-embedding TW61-chol niosome (Invention Product 6) | 0.18 |
| MnT2MPyP | 0.18 |
| MnT4MPyP | 0.43 |
| MnT4MPyP | 0.7*1 |
| *1:1 Batinic-Haberle, L. Benov, and I. Fridovich, J. Biol. Chem., 273, 24251 (1998) | |

The results show that $IC_{50}$ values of MnT2MPyP/1SAS-embedding TW61-chol niosome and MnT2MPyP/4SAS-embedding TW61-chol niosome are close to reported values of low molecular weight MnT2MPyP, indicating that they exhibit an effective SOD activity.

Example 12

Evaluation of SOD activity of Por-embedding niosome (2)

The SOD activity (i.e. $O_2^{-\cdot}$ extinction activity) of Por-embedding niosome was evaluated according to the stopped flow method of Riley et al. (D. P. Riley, W. L. Rivers, and R. H. Weiss, Anal. Biochem., 196, 344(1991)). A specific procedure was as follows: 60 mM HEPES/HEPES Na buffer solutions (8.1 pH) containing Por-embedding niosome at different concentrations were rapidly mixed with a dimethyl sulfoxide solution of potassium superoxide, which is an $O_2^{-\cdot}$ generation source, at 36°C to measure over time an attenuation of absorbance at 245 nm due to $O_2^{-\cdot}$ (a decay curve of $O_2^{-\cdot}$ extinction reaction). The relationship of 1n (absorbance) and time was determined from the decay curve. The apparent rate constant was calculated from the inclination of the resulting relationship. The rate constant ($k_{cat}$) of the $O_2^{-\cdot}$ extinction reaction was then determined from the inclination of the relationship of the metalloporphyrin complex concentration and the apparent rate constant.

Table 6 shows $k_{cat}$ values for various metalloporphyrin complex systems. In the Table, the $k_{cat}$ value of MnT4MPyP is a value disclosed by Ohse, Kawakami, et al. (T. Ohse, S. Nagaoka, Y Arakawa, H. Kawakami, and K. Nakamura, J. Inorg. Biochem., 85, 201 (2001).

Table 6

| Metalloporphyrin complex system | $k_{cat}$ ($M^{-1}s^{-1}$) |
|---|---|
| MnT2MPyP/1SAS-embedding TW61-chol niosome (Invention Product 5) | $3.1 \times 10^7$ |
| MnT2MPyP/4SAS-embedding TW61-chol niosome (Invention Product 6) | $2.0 \times 10^7$ |
| MnT2MPyP | $2.1 \times 10^7$ |
| MnT4MPyP | $1.4 \times 10^7$ |
| MnT4MPyP | $2{,}2 \times 10^7$ *2 |
| *2: T. Ohse, S. Nagaoka, Y Arakawa, H. Kawakami, and K. Nakamura, J. Inorg. Biochem., 85, 201 (2001) | |

The results show that $k_{cat}$ values of MnT2MPyP/1SAS-embedding TW61-chol niosome and MnT2MPyP/4SAS-embedding TW61-chol niosome are close to reported values of low molecular weight MnT2MPyP, indicating that they exhibit an effective SOD activity.

INDUSTRIAL APPLICABILITY

The Por-embedding niosome of the present invention can react with superoxide anionic radicals ($O_2^{-\cdot}$) and can

reduce these radicals without fail.

Therefore, the Por-embedding niosome can exhibit an excellent effect of treating cancer by reducing $O_2^{-\cdot}$ in cancer cells. In addition, since the effect is selective, the Por-embedding niosome can be used as a novel anticancer agent without side effects.

The Por-embedding niosome of the present invention also possesses an SOD activity and can be retained in the blood while exhibiting a superior antioxidation effect. The Por-embedding niosome can thus protect living bodies from hindrance brought about by active oxygen species.

## Claims

1. A metalloporphyrin complex-embedding niosome comprising a cationized metalloporphyrin complex and a niosome-forming substance.

2. The metalloporphyrin complex-embedding niosome according to claim 1, wherein the cationized metalloporphyrin complex forms an ion complex with an anionic surfactant.

3. The metalloporphyrin complex-embedding niosome according to claim 1 or claim 2, wherein the niosome-forming substance is a nonionic surfactant or a mixture of a nonionic surfactant and a cholesterol or a triacylglycerol.

4. The metalloporphyrin complex-embedding niosome according to any one of claims 1 to 3, wherein the niosome is endoplasmic reticula with a diameter of 100 nm or less.

5. The metalloporphyrin complex-embedding niosome according to any one of claims 1 to 4, wherein the cationized metalloporphyrin complex is a complex shown by the following formula (I), (II), or (III),

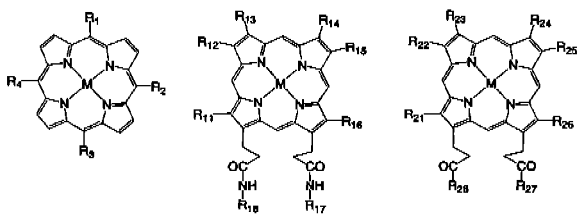

(I) (II) (III)

wherein at least one of $R_1$ to $R_4$ is a group selected from an N-lower-alkylpyridyl group, a lower-alkylammoniophenyl group, and an N-lower-alkylimidazolyl group, $R_{11}$ to $R_{16}$ and $R_{21}$ to $R_{26}$ are lower alkyl or lower alkoxy groups, $R_{17}$ and $R_{18}$ are groups selected from an N-lower-alkylpyridyl group, a lower-alkylammoniophenyl group, and an N-loweralkylimidazolyl group, and $R_{27}$ and $R_{28}$ indicate N-alkylammonio groups.

6. The metalloporphyrin complex-embedding niosome according to any one of claims 1 to 5, wherein the cationized metalloporphyrin complex is one or more of metal[5,10,15,20-tetrakis(2-methylpyridyl)porphyrin] (MT2MPyP), metal [5,10,15,20-tetrakis(4-methylpyridyl)porphyrin] (MT4MPyP), or metal[[1,3,5,8-tetramethyl-2,4-divinyl-6,7-di(4-methylpyridylamideethyl)]porphyrin] (MPPIX-DMPyAm), wherein the metal (M) is iron (Fe), manganese (Mn), cobalt (Co), copper (Cu), molybdenum (Mo), chromium (Cr), or iridium (Ir).

7. The metalloporphyrin complex-embedding niosome according to claim 3, wherein the nonionic surfactant is one or more of polyoxyethylene sorbitan fatty acid esters, sorbitan fatty acid esters, and polyoxyethylene polyoxypropylene copolymers.

8. The metalloporphyrin complex-embedding niosome according to claim 3, wherein the nonionic surfactant is one or more of Tween-61, Tween-80, Span 80, and Pluronic F-88.

9. The metalloporphyrin complex-embedding niosome according to claim 3, wherein the cholesterol is cholesterol, α-cholestanol, β-cholestanol, cholestane, desmosterol (5,24-cholestadiene-3β-ol), sodium cholate, or a cholecalciferol.

10. The metalloporphyrin complex-embedding niosome according to claim 2, wherein the anionic surfactant forming an ion complex with the cationized metalloporphyrin complex is lauric acid, myristic acid, palmitic acid, stearic acid, oleic acid, dodecylsulfuric acid, tetradecylsulfuric acid, hexadecylsulfuric acid, or octadecylsulfuric acid, or a salt of these compounds.

11. A process for producing a metalloporphyrin complex embedding niosome comprising mixing a cationized metalloporphyrin complex and a niosome-forming substance and treating the mixture with supersonic waves in a medium.

12. A process for producing a metalloporphyrin complex-embedding niosome comprising reacting a cationized metalloporphyrin complex and an anionic surfactant to form an ion complex, mixing the resulting ion complex with a niosome-forming substance, and treating the mixture with supersonic waves in a medium.

13. A drug comprising the metalloporphyrin complex-embedding niosome according to any one of claims 1 to 9 as an effective component.

14. The drug according to claim 13, which is an anticancer agent.

15. The drug according to claim 14, which is an antioxidant.

16. The drug according to claim 13, which is a therapeutic agent for inflammation, nervous system diseases, arteriosclerosis, or diabetes.

17. A method of treating cancer comprising administering the metalloporphyrin complex-embedding niosome according to any one of claims 1 to 9 to a cancer patient.

18. The method according to claim 17, wherein the administration is direct dosage, intravenous administration, or subcutaneous administration.

19. A method of treating of inflammation, nervous system disease, arteriosclerosis, or diabetes comprising administering the metalloporphyrin complex-embedding niosome according to any one of claims 1 to 9 to a patient suffering from any one of these diseases.

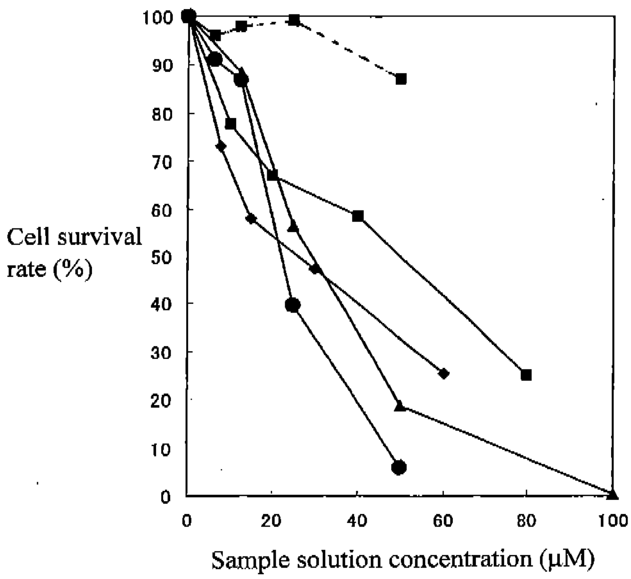
100
90
80
70
60
50
40
30
20
10
0
Cell survival rate (%)
0
20
40
60
80
100
Sample solution concentration (μM)

*FIG. 1*

**INTERNATIONAL SEARCH REPORT**

International application No.
PCT/JP2004/002750

A. CLASSIFICATION OF SUBJECT MATTER
Int.Cl$^7$ A61K31/409, 45/06, A61P3/10, 9/10, 25/00, 29/00, 35/00, 43/00

According to International Patent Classification (IPC) or to both national classification and IPC

B. FIELDS SEARCHED

Minimum documentation searched (classification system followed by classification symbols)
Int.Cl$^7$ A61K31/409, 45/06, A61P3/10, 9/10, 25/00, 29/00, 35/00, 43/00

Documentation searched other than minimum documentation to the extent that such documents are included in the fields searched

| | | | |
|---|---|---|---|
| Jitsuyo Shinan Koho | 1926-1992 | Toroku Jitsuyo Shinan Koho | 1994-1996 |
| Kokai Jitsuyo Shinan Koho | 1971-1992 | Jitsuyo Shinan Toroku Koho | 1996-2004 |

Electronic data base consulted during the international search (name of data base and, where practicable, search terms used)
CA(STN)

C. DOCUMENTS CONSIDERED TO BE RELEVANT

| Category* | Citation of document, with indication, where appropriate, of the relevant passages | Relevant to claim No. |
|---|---|---|
| Y | JP 2000-247978 A (Toyo Ink Manufacturing Co., Ltd.), 12 September, 2000 (12.09.00), Full text (Family: none) | 1-16 |
| Y | JP 5-229948 A (Toyo Jozo Co., Ltd.), 07 September, 1993 (07.09.93), Full text (Family: none) | 1-16 |
| Y | EP 350948 A2 (Toyo Hakko Kogyo Co., Ltd.), 17 January, 1990 (17.01.90), Full text & JP 2-138280 A | 1-16 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 27 April, 2004 (27.04.04) | 18 May, 2004 (18.05.04) |
| Name and mailing address of the ISA/ Japanese Patent Office | Authorized officer |
| Facsimile No. | Telephone No. |

Form PCT/ISA/210 (second sheet) (January 2004)

**INTERNATIONAL SEARCH REPORT**

International application No.
PCT/JP2004/002750

C (Continuation). DOCUMENTS CONSIDERED TO BE RELEVANT

| Category* | Citation of document, with indication, where appropriate, of the relevant passages | Relevant to claim No. |
|---|---|---|
| Y | WO 91/04667 A1 (Rockefeller University), 18 April, 1991 (18.04.91), Full text & JP 4-502019 A | 1-16 |
| Y | WO 98/32463 A2 (Pharmacia & Upjohn Co.), 30 July, 1998 (30.07.98), Full text & JP 2001-511128 A | 1-16 |
| Y | WO 99/02539 A1 (Geange Ltd., Ire.), 21 January, 1999 (21.01.99), Page 14, line 12 & JP 2001-509510 A | 1-16 |
| Y | WO 95/23597 A1 (Trans-Onycha Ltd.), 08 September, 1995 (08.09.95), Claims & JP 9-509676 A | 1-16 |
| Y | US 5882674 A (LTS Lohmann Therapie-Systeme GmbH.), 16 March, 1999 (16.03.99), Claim 8 & JP 10-501539 A | 1-16 |
| Y | US 4830857 A (Oreal S.A.), 16 May, 1989 (16.05.89), Full text & JP 61-178909 A | 1-16 |
| Y | GB 2189457 A1 (Oreal S.A.), 28 October, 1987 (28.10.87), Full text & JP 63-23737 A | 1-16 |

Form PCT/ISA/210 (continuation of second sheet) (January 2004)

**INTERNATIONAL SEARCH REPORT**

International application No.
PCT/JP2004/002750

**Box No. II Observations where certain claims were found unsearchable (Continuation of item 2 of first sheet)**

This international search report has not been established in respect of certain claims under Article 17(2)(a) for the following reasons:

1. [X] Claims Nos.: 17 to 19
because they relate to subject matter not required to be searched by this Authority, namely:
Claims 17 to 19 involve methods for treatment of the human body by surgery or therapy and thus relate to a subject matter which this International Searching Authority is not required, under the provisions of Article 17(2)(a)(i) of the PCT and Rule 39.1(iv) of the Regulations under the PCT, to search.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.

2. [ ] As all searchable claims could be searched without effort justifying an additional fee, this Authority did not invite payment of any additional fee.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** [ ] The additional search fees were accompanied by the applicant's protest.
[ ] No protest accompanied the payment of additional search fees.

Form PCT/ISA/210 (continuation of first sheet (2)) (January 2004)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **K. KALYANASUNDARAM.** *Inorg. Chem.,* 1984, vol. 23, 2453
- **A. D. ADLER et al.** *J. Inorg. Nucl. Chem.,* 1970, vol. 32, 2443
- **T, YONETANI et al.** *J. Biol. Chem.,* 1970, vol. 245, 2988
- **P. HAMBRIGHT.** *Inorg. Chem.,* 1976, vol. 15, 2314
- **E. TSUCHIDA ; H. NISHIDE ; H. YOKOYAMA ; R. YOUNG ; C. K. CHANG.** *Chem. Lett.,* 1984, vol. 991
- **J. M. MCCORD ; I. FRIDOVICH.** *J. Biol. Chem.,* 1969, vol. 244, 6049
- **J. BUTLER ; W. H. KOPENOL ; E. MARGOLIASH.** *J. Biol. Chem.,* 1982, vol. 257, 10747
- **I. BATINIC-HABERLE ; L. BENOV ; I. FRIDOVICH.** *J. Biol. Chem.,* 1998, vol. 273, 24251
- **BATINIC-HABERLE ; L. BENOV ; I. FRIDOVICH.** *J. Biol. Chem.,* 1998, vol. 273, 24251
- **D. P. RILEY ; W. L. RIVERS ; R. H. WEISS.** *Anal. Biochem.,* 1991, vol. 196, 344
- **T. OHSE ; S. NAGAOKA ; Y ARAKAWA ; H. KAWAKAMI ; K. NAKAMURA.** *J. Inorg. Biochem.,* 2001, vol. 85, 201